# EUROPEAN PATENT SPECIFICATION

(11) **EP 3 067 353 B1**
(45) Date of publication and mention of the grant of the patent: **22.11.2017**
(21) Application number: 16156678.1
(22) Date of filing: 29.07.2009
(51) Int. Cl.: C07D 453/02, A61K 31/439, A61P 13/10

(54) **A PROCESS FOR THE PREPARATION OF SOLIFENACIN SALTS AND THEIR INCLUSION INTO PHARMACEUTICAL DOSAGE FORMS**
VERFAHREN ZUR HERSTELLUNG VON SOLIFENACINSALZEN UND EINBAU DAVON IN PHARMAZEUTISCHEN DARREICHUNGSFORMEN
PROCÉDÉ DE PRÉPARATION DE SELS DE SOLIFÉNACINE ET LEUR INCLUSION DANS DES FORMES POSOLOGIQUES PHARMACEUTIQUES

(30) Priority: 29.07.2008 SI 200800188; 20.11.2008 SI 200800285; 21.04.2009 SI 200900113
(43) Date of publication of application: 14.09.2016
(62) Divisional of application: 09777523.3
(73) Proprietor: KRKA, D.D., Novo Mesto, 8501 Novo mesto (SI)
(72) Inventor: RUZIC, Milos, 3000 Celje (SI); PRUDIC, Darja, 8000 Novo mesto (SI); PECAVAR, Anica, 8000 Novo mesto (SI); ZANOTTI-GEROSA, Antonio, Cambridge, CB1 2PY (GB); STROPNIK, Tadej, 8321 Brusnice (SI)
(74) Representative: Andrae | Westendorp Patentanwälte Partnerschaft

(56) References cited:
- WO-A2-2007/076116

## Description

### TECHNICAL FIELD

The present invention relates to potassium 1-phenyl-1,2,3,4-tetrahydroisoquinoline, potassium 1(S)-phenyl-1,2,3,4-tetrahydroisoquinoline, sodium 1-phenyl-1,2,3,4-tetrahydroisoquinoline, sodium 1(S)-phenyl-1,2,3,4-tetrahydroisoquinoline, a process for the preparation of 1-phenyl-1,2,3,4-tetrahydroisoquinoline (II), and a process for the preparation of potassium or sodium 1-phenyl-1,2,3,4-tetrahydroisoquinoline. Furthermore, the present invention relates to the use of 1(S)-phenyl-1,2,3,4-tetrahydroisoquinoline (II) for the preparation of solifenacin or a pharmaceutically acceptable salt thereof, and the use of potassium or sodium 1-phenyl-1,2,3,4-tetrahydroisoquinoline (III) for the preparation of solifenacin or a pharmaceutically acceptable salt thereof.

### BACKGROUND OF THE INVENTION

(1S)-(3R)-1-azabicyclo[2.2.2]oct-3-yl 3,4-dihydro-1-phenyl-2(1*H*)-isoquinoline carboxylate (I) (solifenacin) is used for symptomatic treatment of urgent incontinence and/or increased frequency of urinating and urgency of urinating in patients with a hyperactive urinary bladder. The commercial tablet is marketed under the name Vesicare® and has been approved by the FDA for once daily treatment of OAB in 5 mg and 10 mg tablets. Each Vesicare tablet contains 5 or 10 mg of solifenacin succinate and is formulated for oral administration. In addition to the active ingredient solifenacin succinate, each Vesicare tablet also contains the following inert ingredients: lactose monohydrate, corn starch, hypromellose 2910, magnesium stearate, talc, polyethylene glycol 8000 and titanium dioxide with yellow ferric oxide (5 mg Vesicare tablet) or red ferric oxide (10 mg Vesicare tablet).

One way of the overall preparation of solifenacin base is reported by Mealy, N. et al. in Drugs of the Future 24 (8): 871-874 (1999) is shown in Scheme 1:

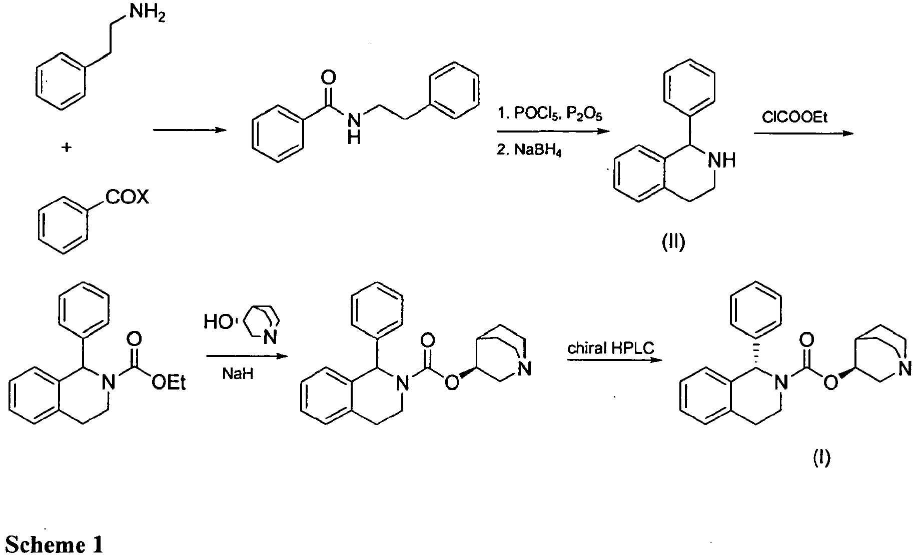

In this approach, optical resolution was performed at the racemic solifenacin base (I) level. Another described method in this article uses tartaric acid for the separation of 1(S)-phenyl-1,2,3,4-tetrahydroisoquinolin (II) from its R-enantiomer, and then reacting it further to yield solifenacin (I).

In EP 0801067 another synthesis of solifenacin is described, wherein 3-quinuclidinyl chloroformate monohydrochloride is reacted with 1(S)-phenyl-1,2,3,4-tetrahydroisoquinolin (II) as shown in Scheme 2:

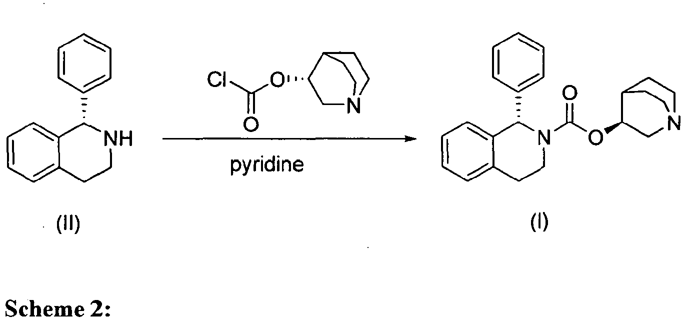

In WO2007/076116 another approach was applied: 1(S)-phenyl-1,2,3,4-tetrahydroisoquinoline (II) and a haloalkyl haloformate were reacted in the presence of a first base to yield a haloalkyl-1,2,3,4-tetrahydroisoquinoline carbamate, which was converted into solifenacin (I) in the next step, as shown by Scheme 3:

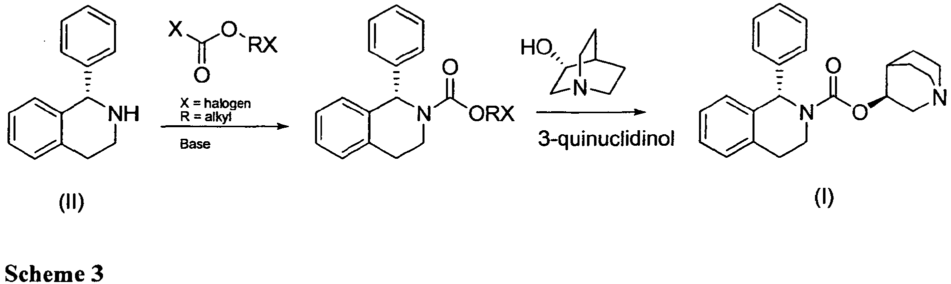

In another embodiment of the same patent application, (R)-3-quinuclidinol is combined with a haloalkyl haloformate in the presence of a base, and in the next step the haloalkyl-quinuclidyl-carbonate obtained is used to obtain solifenacin (Scheme 4):

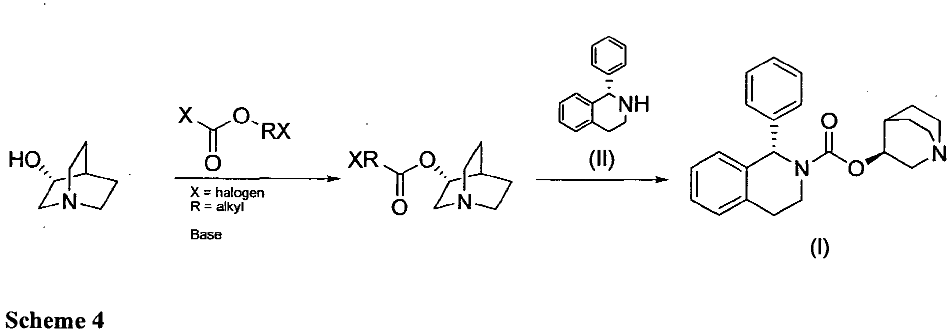

The common feature of all these synthetic approaches is the use of 1(S)-phenyl-1,2,3,4-tetrahydroisoquinoline (II) as the key intermediate. Optical resolution of this intermediate is already reported by Monatshefte fur Chemie, Vol. 53-54: 956-962 (1929) and involves the formation of the tartrate from an aqueous solution. In *J. Med. Chem. Naito et al.* described a similar process using ethanol for the addition of the tartaric acid and crystallization from water. In WO 2008/011462 the solvents for basically the same process were changed to organic solvents, and in WO 2008/019055 the organic solvents used are IPA and EtOAc.

In IPCOM000139416D the use of racemic 1(S)-phenyl-1,2,3,4-tetrahydroisoquinoline oxalate salt (by Rintec Inc.) for the preparation of solifenacin is reported. No further details about the synthesis of solifenacin or any optical resolution are given.

### SUMMARY OF THE INVENTION

Technical problems to be solved by the present invention are:
b.) the provision of an improved method for the preparation of 1(S)-phenyl-1,2,3,4-tetrahydroisoquinoline (II) or its appropriate dichloromethane or toluene solution for further use,
c.) the conversion of 1-phenyl-1,2,3,4-tetrahydroisoquinoline or 1(S)-phenyl-1,2,3,4-tetrahydroisoquinoline into the potassium or sodium salt of 1-phenyl-1,2,3,4-tetrahydroisoquinoline or 1(S)-phenyl-1,2,3,4-tetrahydroisoquinoline (III),
d.) the use of (II) or (III), or the solution of (II) or (III) in dichloromethane or toluene for the synthesis of solifenacin base and pharmaceutically acceptable salts of solifenacin.

As regards the technical problem under b.), it was found out that 1(S)-phenyl-1,2,3,4-tetrahydroisoquinoline (II) is more efficiently prepared by stereoselective/ stereospecific hydrogenation of 1-phenyl-3,4-dihidroisoquinoline (IV) than by optical resolution via diastereoisomeric salts of phenyl-1,2,3,4-tetrahydroisoquinoline (II) and/or may be synthesized on the basis of an alternative synthesis route allowing the use of educts and/or intermediate products as taught by the present invention.

### DESCRIPTION OF THE FIGURES

Fig. 1 shows a powder X-ray diffraction pattern of racemic potassium 1-phenyl-1,2,3,4-tetrahydroisoquinoline (III) (Example 1).

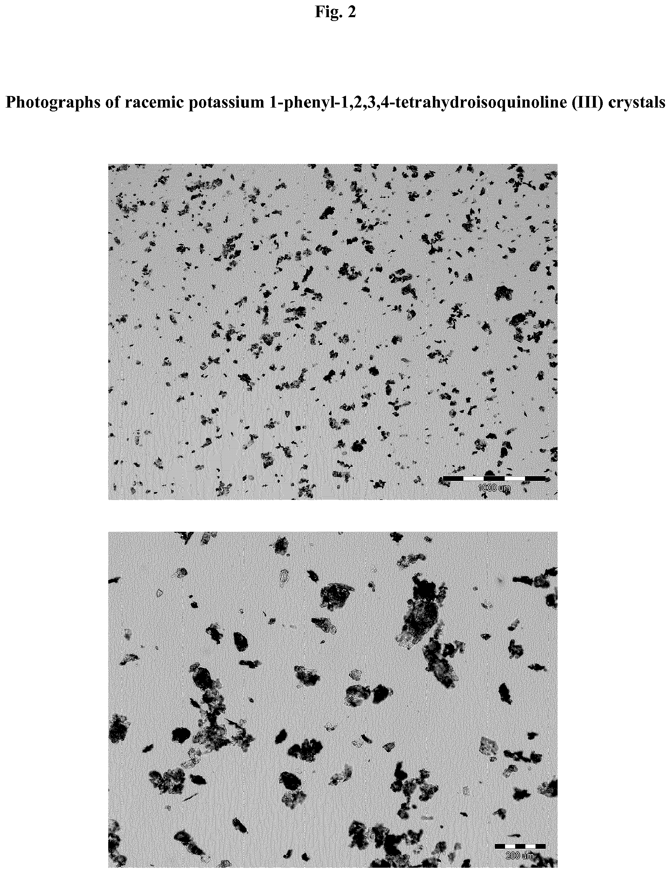

Fig. 2 shows two photographs of racemic potassium 1-phenyl-1,2,3,4-tetrahydroisoquinoline (III) crystals (Example 1).

### DETAILED DESCRIPTION OF THE INVENTION

X-ray powder diffraction patterns were obtained by Phillips PW3040/60 X'Pert PRO diffractometer; CuK_{α} radiation 1,541874 Ǻ.

Racemic potassium 1-phenyl-1,2,3,4-tetrahydroisoquinoline (III) according to the present invention is characterized by the following 2-theta degrees: ±0.2 (Table 15)

**Table 15**

| No. | Pos. [°2Th.] | d-spacing [A] | Rel. Int. [%] |
|---|---|---|---|
| 1 | 10.2 | 8.64 | 96 |
| 2 | 15.3 | 5.77 | 28 |
| 3 | 17.7 | 5.02 | 28 |
| 4 | 18.0 | 4.93 | 30 |
| 5 | 20.6 | 4.32 | 100 |
| 6 | 22.4 | 3.98 | 23 |
| 7 | 24.6 | 3.62 | 24 |
| 8 | 25.8 | 3.45 | 37 |

In the second embodiment of the present invention 1(S)-phenyl-1,2,3,4-tetrahydroisoquinoline (II) is prepared by hydrogenation of 1-phenyl-3,4-dihydroisoquinoline (IV), in particular by stereoselective/ stereospecific hydrogenation of 1-phenyl-3,4-dihydroisoquinoline (IV).

The term "stereoselective/ stereospecific hydrogenation" as used in the present invention encompasses both stereoselective and stereospecific hydrogenations and encompasses in particular stereoselective hydrogenations. The term "stereoselective hydrogenation" comprises in particular hydrogenation reactions wherein among at least two different, at least theoretically possibly resulting stereoisomeric reaction products of the hydrogenation reaction one of said stereoisomers is exclusively formed or obtained in higher yields than the other stereoisomer(s), in particular is formed in an amount of at least 55 wt.-%, preferably at least 75 wt.-% more preferably at least 90 wt.-%, based on the total weight of all stereoisomeric reaction products resulting from the same educt.

The term "optically enriched 1-phenyl-1,2,3,4-tetrahydroisoquinoline (or salt thereof)" in particular encompasses that 1(S)-phenyl-1,2,3,4-tetrahydroisoquinoline (or salt thereof) is present in a mixture of 1-phenyl-1,2,3,4-tetrahydroisoquinoline (or salt thereof) stereoisomers in an amount of more than 50 wt.%, preferably of more than 70 wt.-%, more preferably of more than 90 wt.-%, based on the total weight of all stereosisomers of 1-phenyl-1,2,3,4-tetrahydroisoquinoline (or salt thereof). The term "optically pure 1(S)-phenyl-1,2,3,4-tetrahydroisoquinoline (or salt thereof)" may in particular mean that 1(S)-phenyl-1,2,3,4-tetrahydroisoquinoline (or salt thereof) is present in a mixture of 1-phenyl-1,2,3,4-tetrahydroisoquinoline (or salt thereof) stereoisomers in an amount of more than 97 wt.%, preferably of more than 99 wt.-%, more preferably of 100 wt.-%, based on the total weight of all stereosisomers of 1-phenyl-1,2,3,4-tetrahydroisoquinoline (or salt thereof).

The hydrogenation reaction, in particular the stereoselective/ stereospecific hydrogenation, is accomplished by reacting 1-phenyl-3,4-dihydroisoquinoline (IV) with a hydrogenating agent in the presence of a hydrogenation reaction catalyst, in particular in the presence of organometallic molecular catalyst comprising a metal atom or metal ion having one or more chiral ligands coupled thereto, further in particular in the presence of an asymmetric organometallic molecular catalyst comprising a metal atom or ion having one or more chiral ligands coupled thereto.

In terms of preferred process parameters, the hydrogenating agent is preferably H₂ and/or the hydrogenating step is carried out at a pressure of from about 3 bar to 100 bar, preferably from about 5 bar to 40 bar, and at a temperature of from about 5 °C to 70 °C, preferably from 40 to 65 °C. The reaction is normally allowed to proceed for a period of from about 2 hours to 7 days. Typically, the reaction mixture is agitated during the hydrogenating step.

The active catalyst can be prepared from a Ti (IV), Pd(II), Ir, Pt (IV), Rh or Ru (IV) salt under the action of reducing agents, preferably Ir catalysts are used. The active catalyst may be prepared in advance, optionally deposited on a catalyst support material such as silica, alumina, carbon, titanium dioxide, molecular sieves, zeolites, kieselguhr, etc., or generated in the reaction mixture. Preferably the active catalyst is prepared in situ.

It was observed by the present invention that the presence of additives such as 1,4-diazabicyclo[2.2.2]octane (DABCO), NaI, MgI₂, Bu₄NI, Bu₄NBr, pyperazine, H₃PO₄, acetic acid, p-toluene sulphonic acid, HBF₄, HCl, HCOOH, or NaH₂PO₄ in amounts between 0.1 and 2 molar equivalents, preferably between 1 and 2 molar equivalents with regard to the substrate leads to better conversion and higher yields.

The catalyst used in the hydrogenation reaction is preferably one or more of the well-known Noyori catalysts such as RuBr₂(S)-binap, [(R,R)-ethylene bis(4,5,6,7-tetrahydro-1-indenyl) titanium (IV)] (R )-1,1'-binaphtyl-2,2'-diolate, Ir-Meo-Biphep, Ir-P-Phos, Ir-Binap, Ir-biphosphonite, 5-Ts, or 5-Nps. 5-Ts and 5-Nps, respectively have the structural formulas shown below, wherein Me stands for methyl:

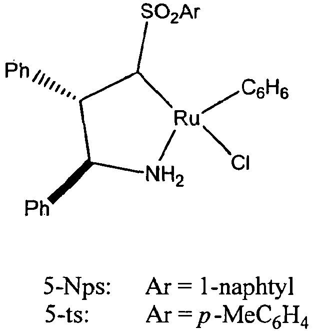

The abbreviation Binap stands for 2,2'-bis(diphenylphosphino)-1,1'-binapthalene, the abbreviation Meo-Biphep stands for (6,6'-dimethoxybiphenyl-2,2'-diyl)-bis(diphenylphosphine), the abbreviation P-Phos stands for 2,2',6,6'-tetramethoxy-4,4'-bis(diphenylphosphino)-3,3'-bipyridine.

The catalysts, in particular the stereoconfiguration of the catalyst and of the ligands thereof, can be chosen or optimized on the basis of standard experiments carried out by a skilled person and on the basis of the teachings of the present application, in particular as given in the Example section.

However, a variety of other catalysts of this type can also be employed. The catalyst is generally used at a level of from about 5 - 25 mol % in the reaction mixture, with respect to the molar amount of 1-phenyl-3,4-dihydroisoquinoline.

In another preferred embodiment, (S)-P-Phos-iridium and/or (S)-Binap-iridium can be used. (S)-P-Phos-iridium and/or (S)-Binap-iridium can be either pre-prepared or prepared in situ, from [Ir COD Cl] and a slight excess (1.15 to 1.50 equivalent to 1 equivalent of Ir) of the ligand, preferably in the presence of the substrate and optionally in the presence of an additive, preferably H₃PO₄. (The abbreviation COD stands for cis, cis-1,5-cyclooctadien.) The temperature is preferably between 55 to 70°C, most preferably between 55°C and 60°C, and the hydrogen pressure can be 10 to 30 bar, preferably between 18 and 22 bars. The catalyst loadings used may be between 100/1 to 3000/1 S/C (molar substrate to catalyst ratio). As solvents preferably tetrahydrofurane, isopropanol, dichloromethane, toluene, 2-methyltetrahydrofurane may be used. Most preferably THF is applied.

The solvents for the coupling reaction can be chosen on the basis of the general knowledge in the art, and in particular can be selected from a variety of known process solvents. Illustrative of the coupling solvents that can be utilized either singly or in combinations are benzene, toluene, tetrahydrofuran, dioxane, acetonitrile, dimethylformamide, dimethylacetamide, ethanol, methanol, propanol, water, dichloroethane, 2-methyltetrahydrofuran or diethoxymethane, N-methylpyrrolidinone, hexamethylphosphoramide, supercritical CO₂, and/or ionic liquids. Preferably tetrahydrofurane is used.

The produced 1(S)-phenyl-1,2,3,4-tetrahydroisoquinoline, in particular produced as set forth above, according to one embodiment can be converted to the potassium or sodium salt of 1(S)-phenyl-1,2,3,4-tetrahydroisoquinoline (III) by dissolving 1(S)- (II) in a suitable solvent, preferably water, and by adding a potassium or sodium base, preferably KOH and/or NaOH, or 1(S)-phenyl-1,2,3,4-tetrahydroisoquinoline (II) or its salt (III) may be used in the next step without isolation from the final purification step in dichloromethane or toluene, in form of a solution. Analogously also the potassium and sodium salt of racemic 1-phenyl-1,2,3,4-tetrahydroisoquinoline are prepared.

In the frame of the present invention, the term "1-phenyl-1,2,3,4-tetrahydroisoquinoline (II) and salts of 1-phenyl-1,2,3,4-tetrahydroisoquinoline (III)" encompasses inter alia in particular 1(S)-phenyl-1,2,3,4-tetrahydroisoquinoline and salts thereof, as well as racemic 1-phenyl-1,2,3,4-tetrahydroisoquinoline and salts thereof.

According to another aspect, the present invention provides a solution of 1(S)-phenyl-1,2,3,4-tetrahydroisoquinoline (II) and/or its sodium and/or potassium salt(s) (III) in toluene and/or in dichloromethane, as well as a solution of a mixture of 1(S)-phenyl-1,2,3,4-tetrahydroisoquinoline and 1(R)-phenyl-1,2,3,4-tetrahydroisoquinoline and/or the sodium and/or potassium salt(s) (III) thereof in toluene and/or dichloromethane.

The term "solution of a compound (such as e.g. 1(S)-phenyl-1,2,3,4-tetrahydroisoquinoline) in a specified solvent or a specified mixture of solvents (such as e.g. toluene)" encompasses in the frame of the present invention in particular any mixture comprising the compound and the specified solvent / the specified mixture of solvents, and encompasses preferably any mixture wherein the total weight of the compound and the specified solvent / mixture of solvents is at least 80 wt.%, more preferably at least 95 wt.%, or in particular 100 wt.-%, based on the total weight of the mixture comprising the compound and the specified solvent / mixture of solvents. Furthermore said term "solution of a compound in a specified solvent or a specified mixture of solvents" may encompass also suspensions and emulsions. For many technical applications solutions free of the non-dissolved compound and in particular free of any not dissolved material can be preferred.

In order to complete the reaction sequence, the 1(S)-phenyl-1,2,3,4-tetrahydroisoquinoline or its potassium or sodium salt resulting from the hydrogenation reaction, in particular the Noyori reduction is reacted with ethyl chloroformiate and converted to solifenacin base (I). Solifenacin free base (I) can than be converted into an acid addition salt and incorporated into a pharmaceutical formulation as described above.

1 (S)-phenyl-1,2,3,4-tetrahydroisoquinoline and its impurities were determined by means of a HPLC method, comprising:

### Chromatographic conditions:

**Column:** Ascentis Express C8, 2.7 µm particles, 100 x 4.6 mm i.d. The following experimental data were obtained using the before-mentioned Ascentis Express C8 column. (Another equivalent column, filled with ODS reverse phase can also be used. If necessary, adjust slightly flow rate and/or the time gradient of the mobile phase to assure the chromatographic system suitability.)

| **Mobile Phase:** | Gradient elution |
|---|---|
| | A: 0.02 M NaH₂PO₄, pH 2.5 |
| | B: acetonitrile and methanol in the ratio 50:50 |

### Gradient:

| Time (min) | % A | % B |
|---|---|---|
| 0 | 75 | 25 |
| 3.5 | 75 | 25 |
| 16.5 | 0 | 100 |
| 18.5 | 0 | 100 |
| 19.5 | 75 | 25 |

Filter the mobile phases A and B through a 0.22 µm membrane filter and degas it (by ultrasound or helium) prior to use.

| | |
|---|---|
| **Post run:** | 3 min |
| **Column temp:** | 25°C |
| **Flow-rate:** | 1.0 ml/min |
| **Detection:** | UV, wavelength 210 nm |
| **Injection:** | 5 µl |

Solifenacin, its salts and their impurities were determined by means of a second HPLC method, comprising:

### Chromatographic conditions

**Column:** Ascentis Express C8, 2.7 µm particles, 100 x 4.6 mm i.d., The following experimental data were obtained using the before-mentioned Ascentis Express C8 column.

(Another equivalent column, filled with ODS reverse phase can also be used. If necessary, adjust slightly flow rate and/or the time gradient of the mobile phase to assure the chromatographic system suitability.)

| **Mobile phase:** | gradient elution |
|---|---|
| | A: 0.02M NaH₂PO₄ pH 2.5 |
| | B: acetonitrile |
| | C: methanol |

### Gradient:

| Time (min) | % A | %B | %C |
|---|---|---|---|
| 0 | 75 | 12.5 | 12.5 |
| 3.5 | 75 | 12.5 | 12.5 |
| 16.5 | 0 | 50 | 50 |
| 18.5 | 0 | 50 | 50 |
| 20 | 0 | 87.5 | 12.5 |
| 23 | 0 | 87.5 | 12.5 |
| 25 | 75 | 12.5 | 12.5 |

Filter the mobile phases A and B through a 0.22 µm membrane filter and degas it (by ultrasound or helium) prior to use.

| | |
|---|---|
| **Post run:** | 3 min |
| **Column temp:** | 25°C |
| **Flow-rate:** | 0.7 ml/min |
| **Detection:** | UV, wavelength 210 nm |
| **Injection:** | 5 µl |

The invention is explained specifically by the following examples which are not intended as a limitation thereof and are not intended to restrict the scope of the invention. Unless explicitly indicated otherwise, the term "room temperature" means 20°C.

### EXAMPLES

### Example 1

### Preparation of 1(S)-phenyl-1,2,3,4-tetrahydroisoquinoline (II)

### Procedure A:

118 g of [(R,R)-ethylene bis(4,5,6,7-tetrahydro-1-indenyl) titanium (IV)] (R )-1,1'-binaphtyl-2,2'-diolate, 20 mL of THF, 242 µL BuLi and 74 µL phenylsylane were mixed at room temperature in nitrogen atmosphere, and 0.82 g of 1-phenyl-3,4-dihydroisoquinoline in 4 mL THF was added to the suspension. The reaction mixture was then transported to the hydrogenation reactor Hel Automate II. Hydrogenation started at room temperature and proceeded at 65 °C at a pressure between 5 and 100 bar until the reaction was concluded. After the reaction was completed, the solvent fractions were evaporated *under vacuum* and the product was re-crystallized from diethyl ether.
In the product isolated the ratio between the R and the S isomer was 9:91.

### Procedure B1:

0.0025 mmol [IrCOD)Cl]₂, 0.0055 mmol (S)-BINAP and 0.25 mmol of 1-phenyl-3,4-dihydroisoquinoline were weighted in a glass liner, placed in the hydrogenation reactor and then under inert atmosphere 3 mL of THF was added. The reaction was purged with hydrogen, heated to 50°C and hydrogen pressure topped up to 30 bar. After 3 hours the yield of the product was 85% and the enantiomeric excess was 80 % (S).

If additives were used, the following results were obtained under identical reaction conditions:

| **Additive** | **Conversion** | **Enantiomeric excess of 1(S)-phenyl-1,2,3,4-tetrahydroisoquinoline (II)** |
|---|---|---|
| NaI, 0.2 mol. eq. | 92% | 55% |
| MgI₂, 0.2 mol. eq. | 84% | 46% |
| H₃PO₄ (anhydrous), 2 eq. | 98% | 82% |
| H₃PO₄ (anhydrous), 1 eq. | 98% | 87% |
| H₃PO₄ (85% w/w in water), 1 eq. | 98% | 87% |
| AcOH 1 eq. (AcOH in THF) | 53% | 72% |
| AcOH 10 eq. (AcOH in THF) | 89% | 78% |

### Procedure B2:

0.0025 mmol [IrCOD)Cl]₂ and 0.0055 mmol (S)-BINAP were weighted in a glass liner, placed in the hydrogenation reactor and then under inert atmosphere 3 mL of THF was added. The reaction was stirred at room temperature for 30 minutes and then a solution of 0.25 mmol of 1-phenyl-3,4-dihydroisoquinoline was added. 2 mL of dichloroethane was added to solubilize 1-phenyl-3,4-dihydroisoquinoline. The reaction was purged with hydrogen, heated to 50°C and hydrogen pressure topped up to 30 bar. After 3 hours the yield of the product was over 97% and the enantiomeric excess was 50 % (S).

### Procedure C:

[IrCODCl]₂ (0.05 mmol, 33.5 mg), (S)-P-Phos (0.115 mmol, 1.15 eq to Ir, 74 mg), 1-phenyl-3,4-dihydroisoquinoline (100 mmol, 20.7 g) were placed in a steel 600 mL Parr autoclave (equipped with overhead stirrer, internal temperature probe, external heating jacket). The autoclave was sealed and placed under nitrogen. THF (200 mL, anhydrous grade, Aldrich) and H₃PO₄ (85% in water, 10.3 mL, 150 mmol) were pre-mixed (mild exotherm) in air and then added to the solid in the autoclave using a 50 mL syringe through the autoclave injection port. The autoclave was sealed and then purged with nitrogen five times by pressurizing to 3 bar under stirring and then releasing pressure. The reaction was then purged with hydrogen five times by pressurizing to 30 bar under stirring and then releasing pressure. The pressure was set to 20 bar and the reaction was heated to 60°C over 30 minutes (pressure increased to about 22 bar). The reaction was stirred at maximum stirring speed (1200-1500 rpm) for 68 hours, then it was cooled to 45°C, opened to find a clear, thick yellow/green solution that was sampled. Analysis on AD-H column showed full conversion to amine (II) and 95% ee (S).

Work up: The reaction was diluted with MeOH (200 mL),l transferred to a 500 mL roundbottomed flask and concentrated under reduced pressure to obtain a clear yellow/green oil. Dichloromethane (100 mL) was added, followed by 50 mL of water and 60 mL of 25% ammonium hydroxide (to constant pH 8-9). The layers were separated and the aqueous layer was further extracted with dichloromethane (4x 100 mL). The organic layers were combined, dried over Na₂SO₄, the filter cake was washed with more dichloromethane (2 x 75 mL) and the dichloromethane solution was evaporated under reduced pressure to give an off-white solid (more than 99% amine (II), 94% ee, 18.5 g, 88.5% yield), or can be used directly further in the synthesis of solifenacin carbamate.

### Preparation of sodium 1(S)-phenyl-1,2,3,4-tetrahydroisoquinoline (II)

0.3 g (1.43 mmol) 1 (S)-phenyl-1,2,3,4-tetrahydroisoquinoline is mixed with 6 mL of demineralised water and 0.06 g (1.43 mmol) of NaOH (solid). The suspension is agitated at room or higher temperature (e.g. 50 °C), mixed there for some time and cooled to temperatures above 0 °C. The product is filtered, washed with water and dried. 0.22 g of sodium salt is formed.

### Preparation of potassium 1-phenyl-1,2,3,4-tetrahydroisoquinoline (II)

0.3 g (1.43 mmol) 1-phenyl-1,2,3,4-tetrahydroisoquinoline is mixed with 6 mL demineralised water and 0.08 g (1.43 mmol) of KOH (solid). The suspension is agitated at room or higher temperature (e.g. 50 °C), mixed there for some time and cooled to temperatures above 0 °C. The product is filtered, washed with water and dried. 0.24 g of potassium salt is formed.

### Example 2

### Preparation of solifenacin carbamate

To a solution of 5 g of 1(S)-phenyl-1,2,3,4-tetrahydroisoquinoline in 50 mL dichloromethane, 6.6 g of potassium carbonate were added, the mixture was agitated and cooled to 0 - 5 °C. 4.55 mL of ethyl chloroformate were slowly added to the mixture and the white emulsion obtained was stirred at room temperature over night. 50 mL of demineralized water were added and the mixture was stirred for further 30 minutes. The organic phase was separated and concentrated until a colorless oil (6 g) was obtained. HPLC chromatographic purity was 99.8 area %.

### Preparation of solifenacin carbamate

To a solution of 100 g of 1(S)-phenyl-1,2,3,4-tetrahydroisoquinoline in 500 mL dichloromethane, 99 g of potassium carbonate were added, agitated and cooled to 0 - 5 °C. 91 mL of ethyl chloroformate were slowly added and the white emulsion obtained was stirred at room temperature over night. 400 mL of demineralized water were added and the mixture was stirred for further 30 minutes. The organic phase was separated and concentrated until a colorless oil (114 g) was obtained. HPLC chromatographic purity was 98.3 area %, ee purity was 99.97 area %.

### Preparation of solifenacin carbamate

To a solution of 5 g of 1(S)-phenyl-1,2,3,4-tetrahydroisoquinoline in 25 mL dichloromethane (Ph = 10.3), agitated and cooled to 0 - 5 °C, 1.2 mL of ethyl chloroformate were slowly added. Then solutions of 1.2 mL of ethyl chloroformate in 10 ml of dichloromethane and a solution of 3.5 potassium carbonate in 10 mL of demineralised water were added to reaction mixture in parallel. pH was controlled (regulated by speed of addition either of solutions) to be between 8 and 9. The mixture was further agitated for 15 minutes at 5° C and then heated in 40 minutes to room temperature, at which it was agitated for 40 minutes. The organic phase was separated, washed with water, dried with Na₂SO₄ and concentrated until a colorless oil (5.3 g) was obtained. HPLC chromatographic purity was 98.7 area %, ee purity was 99.91 area %.

### Preparation of solifenacin carbamate

To solution of 100 g of 1(S)-phenyl-1,2,3,4-tetrahydroisoquinoline in 700 mL dichloromethane, agitated and cooled to 0 - 5 °C, solution of 27,3 mL of ethyl chloroformate in 284 g dichloromethane were slowly (30 minutes) added. Then solutions of 27.3 mL of ethyl chloroformate in 284 g of dichloromethane and solution of 70g potassium carbonate in 500 mL of demineralised water were added to reaction mixture in parallel. The pH value was controlled (regulated by speed of addition either of solutions) to be between 8 and 9. The mixture was further agitated for 15 minutes at 5 °C and then heated in 10 minutes to room temperature, at which it was agitated for 60 minutes. The organic phase was separated, washed with 100 ml of water and concentrated until a colorless oil (135.7 g) was obtained. HPLC chromatographic purity was 99.8 area %.

### Preparation of solifenacin carbamate

To a solution of 20 g of 1(S)-phenyl-1,2,3,4-tetrahydroisoquinoline in 100 mL toluene, 19.81 g of potassium carbonate were added, agitated and cooled to 0 - 5 °C. 10 mL of ethyl chloroformate were slowly added and the white emulsion obtained was stirred at room temperature over night. 100 mL of demineralized water were added and the mixture was stirred for further 30 minutes. The organic phase was separated and concentrated until a colorless oil (27,5 g) was obtained. HPLC chromatographic purity was 94.4 area %, ee purity was 99.96 area %.

### Preparation of solifenacin carbamate

5 g of (S)-1-phenyl-1,2,3,4-tetrahydroisoquinoline were dissolved in 25 mL of dichloromethane and cooled to 0°C. During the addition of 2 mL of methyl chloroformate the final pH was 0.9 at 0°C. After the addition of the first 2 mL of methyl chloroformate, another 2 mL of methyl chloroformate were dissolved in 10 mL dichloromethane, and the solution of 3.5 g of potassium carbonate in 25 mL of water was added to the reaction mixture in parallel. During the addition pH of suspension is measured and it was controlled (by addition speed of both solutions) to be in the interval between 8 and 9. The reaction mixture was left stirring for 15 minutes, after which the reaction mixture was slowly warmed to room temperature. After stirring for 40 minutes at room temperature, the two phases of the reaction mixture were separated and the organic phase is washed with demineralized water and dried over anhydrous sodium sulfate (VI). The volatile components were evaporated *in vacuo.* The purity of the isolated product obtained was 98.6% (HPLC).

### Preparation of solifenacin base (I)

24 g of solifenacin ethyl carbamate were dissolved in 170 mL of toluene in inert atmosphere and (optionally) water was removed by azeotrope distillation (removed toluene was replaced with fresh toluene). Then 3.4 g of NaH (n(solifenacin ethyl carbamate) : n((R) -quinuclidin-3-ol : n(NaH) = 1 : 3 : 1; NaH could be 60 % moistened with oil or > 95 % dry - Sigma-Aldrich) were added together with 32.5 g of (R) -quinuclidin-3-ol to reaction mixture. The mixture was heated under reflux for at least 3 h, toluene/water azeotrope was continuously removed and replaced with fresh toluene. A yellow solution was formed which was cooled to room temperature and left stirring overnight in inert atmosphere. Reaction mixture was slowly transferred to 100 mL brine (NaCl solution), the phases were separated and 100 mL of 15 % HCl solution were added to organic phase. The phases were again separated. To the aqueous phase 80 mL of 20 % NaOH were added, the pH value was adjusted to alkaline and 100 mL of ethylacetate were then mixed with this solution. The phases were separated and the organic phase(s) (the aqueous phase could be extracted with additional portion of ethylacetate) are dried over sodium sulfate (VI) and the volatile fraction was evaporated *under vacuum.* 29 g of the product was obtained. More than 75 area % HPLC purity and more than 99.5 area % ee purity was obtained.

### Preparation of solifenacin base (I)

20.1 g of solifenacin ethyl carbamate and 10.9 g (R) -quinuclidin-3-ol were dissolved in 100 mL of toluene under inert atmosphere and (optionally) water was removed by azeotrope distillation (removed toluene was replaced with fresh one). Then 1.1 g of NaH (n(solifenacin ethyl carbamate) : n((R) -quinuclidin-3-ol : n(NaH) = 1 : 1,2 : 0,3; NaH could be 60 % moistened with oil or > 95 % dry - Sigma-Aldrich) were added to reaction mixture. The mixture was heated under reflux for at least 3 h, toluene/water azeotrope was continuously removed and replaced with fresh toluene. A yellow solution was formed which was cooled to room temperature and left stirring overnight in inert atmosphere. Reaction mixture was slowly transferred to 120 mL brine (NaCl solution), phases were separated and organic phase was washed twice with 150 mL of water. The organic phase was dried over anhydrous sodium sulfate (VI) and the volatile fraction was evaporated *under vacuum.* 24.9 g of the product was obtained. More than 93 area % HPLC purity and more than 99.9 area % ee purity was achieved.

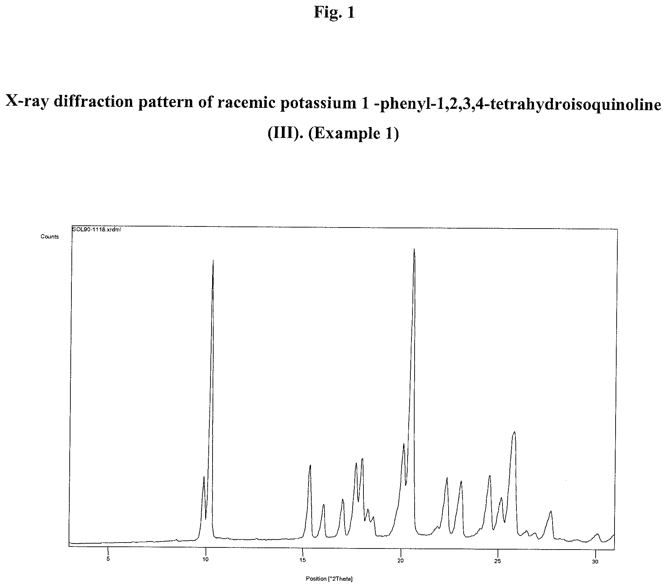

In particular, the present invention provides the following items:
47. Potassium 1-phenyl-1,2,3,4-tetrahydroisoquinoline.
48. Potassium 1 (S)-phenyl-1,2,3,4-tetrahydroisoquinoline.
49. Potassium 1-phenyl-1,2,3,4-tetrahydroisoquinoline according to item 47, characterized by the X-ray powder diffraction pattern as shown in Figure 1.
50. Potassium 1-phenyl-1,2,3,4-tetrahydroisoquinoline according to items 47 and 49, characterized by the following 2-theta degrees: 10.2, 15.3, 17.7, 18.0, 20.6, 22.4, 24.6, 25.8 ±0.2.
51. Sodium 1-phenyl-1,2,3,4-tetrahydroisoquinoline.
52. Sodium 1(S)-phenyl-1,2,3,4-tetrahydroisoquinoline.
53. A process for the preparation of 1-phenyl-1,2,3,4-tetrahydroisoquinoline (II), in particular optically enriched or optically pure 1(S) -phenyl-1,2,3,4-tetrahydroisoquinoline (II), characterized in that 1-phenyl-3,4-dihydroisoquinoline (IV) is reduced with a hydrogenating agent in the presence of a catalyst, in particular an asymmetric organometallic molecular catalyst comprising a metal atom or ion having one or more chiral ligands coupled thereto.
54. A process for the preparation of potassium or sodium 1-phenyl-1,2,3,4-tetrahydroisoquinoline, in particular optically enriched or pure potassium or sodium 1(S) - phenyl-1,2,3,4-tetrahydroisoquinoline, characterized in that the product of the process of item 53 is transformed into the potassium or sodium salt.
55. Use of 1(S)-phenyl-1,2,3,4-tetrahydroisoquinoline (II), in particular optically enriched or optically pure 1(S) -phenyl-1,2,3,4-tetrahydroisoquinoline (II), obtainable according to the process of item 53 for the preparation of solifenacin or a pharmaceutically acceptable salt thereof.
56. Use of potassium or sodium 1-phenyl-1,2,3,4-tetrahydroisoquinoline (III), in particular optically enriched or optically pure potassium or sodium 1(S) -phenyl-1,2,3,4-tetrahydroisoquinoline (III) for the preparation of solifenacin or a pharmaceutically acceptable salt thereof.

## Claims

1. Potassium 1-phenyl-1,2,3,4-tetrahydroisoquinoline.

2. Potassium 1 (S)-phenyl-1,2,3,4-tetrahydroisoquinoline.

3. Potassium 1-phenyl-1,2,3,4-tetrahydroisoquinoline according to claim 1, **characterized by** the X-ray powder diffraction pattern as shown in Figure 1.

4. Potassium 1-phenyl-1,2,3,4-tetrahydroisoquinoline according to claims 1 and 3, **characterized by** the following 2-theta degrees: 10.2, 15.3, 17.7, 18.0, 20.6, 22.4, 24.6, 25.8 ±0.2.

5. Sodium 1-phenyl-1,2,3,4-tetrahydroisoquinoline.

6. Sodium 1(S)-phenyl-1,2,3,4-tetrahydroisoquinoline.

7. A process for the preparation of 1-phenyl-1,2,3,4-tetrahydroisoquinoline (II), in particular optically enriched or optically pure 1(S) -phenyl-1,2,3,4-tetrahydroisoquinoline (II), **characterized in that** 1-phenyl-3,4-dihydroisoquinoline (IV) is reduced with a hydrogenating agent in the presence of a catalyst, in particular an asymmetric organometallic molecular catalyst comprising a metal atom or ion having one or more chiral ligands coupled thereto.

8. A process for the preparation of potassium or sodium 1-phenyl-1,2,3,4-tetrahydroisoquinoline, in particular optically enriched or pure potassium or sodium 1(S) - phenyl-1,2,3,4-tetrahydroisoquinoline, **characterized in that** the product of the process of claim 7 is transformed into the potassium or sodium salt.

9. Use of 1(S)-phenyl-1,2,3,4-tetrahydroisoquinoline (II), in particular optically enriched or optically pure 1(S) -phenyl-1,2,3,4-tetrahydroisoquinoline (II), obtainable according to the process of claim 7 for the preparation of solifenacin or a pharmaceutically acceptable salt thereof.

10. Use of potassium or sodium 1-phenyl-1,2,3,4-tetrahydroisoquinoline (III), in particular optically enriched or optically pure potassium or sodium 1(S) -phenyl-1,2,3,4-tetrahydroisoquinoline (III), for the preparation of solifenacin or a pharmaceutically acceptable salt thereof.

## Patentansprüche

1. Kalium-1-phenyl-1,2,3,4-tetrahydroisochinolin.

2. Kalium-1(S)-phenyl-1,2,3,4-tetrahydroisochinolin.

3. Kalium-1-phenyl-1,2,3,4-tetrahydroisochinolin gemäß Anspruch 1, charakterisiert durch das Röntgenpulverbeugungsmuster wie in Figur 1 gezeigt.

4. Kalium-1-phenyl-1,2,3,4-tetrahydroisochinolin gemäß den Ansprüchen 1 und 3, charakterisiert durch die folgenden 2-Theta Grade: 10,2, 15,3, 17,7, 18,0, 20,6, 22,4, 24,6, 25,8 ± 0,2.

5. Natrium-1-phenyl-1,2,3,4-tetrahydroisochinolin.

6. Natrium-1(S)-phenyl-1,2,3,4-tetrahydroisochinolin.

7. Verfahren zur Herstellung von 1-Phenyl-1,2,3,4-tetrahydroisochinolin (II), insbesondere optisch angereichertem oder optisch reinem 1(S)-Phenyl-1,2,3,4-tetrahydroisochinolin (II), **dadurch gekennzeichnet, dass** 1-Phenyl-3,4-dihydroisochinolin (IV) mit einem Hydrierungsmittel in der Gegenwart eines Katalysators, insbesondere eines asymmetrischen organometallischen molekularen Katalysators, umfassend ein Metallatom oder -ion mit einem oder mehr daran gekuppelten chiralen Liganden, reduziert wird.

8. Verfahren zur Herstellung von Kalium- oder Natrium-1-phenyl-1,2,3,4-tetrahydroisochinolin, insbesondere optisch angereichertem oder reinem Kalium- oder Natrium-1(S)-phenyl-1,2,3,4-tetrahydroisochinolin, **dadurch gekennzeichnet, dass** das Produkt des Verfahren von Anspruch 7 in das Kalium- oder Natriumsalz umgewandelt wird.

9. Verwendung von 1(S)-Phenyl-1,2,3,4-tetrahydroisochinolin (II), insbesondere optisch angereichertem oder optisch reinem 1(S)-Phenyl-1,2,3,4-tetrahydroisochinolin (II), das gemäß dem Verfahren von Anspruch 7 erhalten werden kann, zur Herstellung von Solifenacin oder einem pharmazeutisch verträglichen Salz davon.

10. Verwendung von Kalium- oder Natrium-1-phenyl-1,2,3,4-tetrahydroisochinolin (III), insbesondere optisch angereichertem oder optisch reinem Kalium- oder Natrium-1(S)-phenyl-1,2,3,4-tetrahydroisochinolin (III), zur Herstellung von Solifenacin oder einem pharmazeutisch verträglichen Salz davon.

## Revendications

1. 1-Phényl-1,2,3,4-tétrahydroisoquinoline potassique.

2. 1(S)-Phényl-1,2,3,4-tétrahydroisoquinoline potassique.

3. 1-Phényl-1,2,3,4-tétrahydroisoquinoline potassique selon la revendication 1, **caractérisée par** le spectre de diffraction des rayons X sur poudre tel qu'illustré sur la Figure 1.

4. 1-Phényl-1,2,3,4-tétrahydroisoquinoline potassique selon les revendications 1 et 3, **caractérisée par** les degrés 2-thêta suivants : 10,2, 15,3, 17,7, 18,0, 20,6, 22,4, 24,6, 25,8 ± 0,2.

5. 1-Phényl-1,2,3,4-tétrahydroisoquinoline sodique.

6. 1(S)-phényl-1,2,3,4-tétrahydroisoquinoline sodique.

7. Procédé de préparation de 1-phényl-1,2,3,4-tétrahydroisoquinoline (II), en particulier de 1(S)-phényl-1,2,3,4-tétrahydroisoquinoline (II) optiquement enrichie ou optiquement pure, **caractérisé en ce que** la 1-phényl-3,4-dihydroisoquinoline (IV) est réduite à l'aide d'un agent d'hydrogénation en présence d'un catalyseur, en particulier d'un catalyseur moléculaire organométallique asymétrique comprenant un atome ou un ion de métal comportant un ou plusieurs ligands chiraux couplés à celui-ci.

8. Procédé de préparation de 1-phényl-1,2,3,4-tétrahydroisoquinoline potassique ou sodique, en particulier de 1(S)-phényl-1,2,3,4-tétrahydroisoquinoline potassique ou sodique optiquement enrichie ou pure, **caractérisé en ce que** le produit du procédé selon la revendication 7 est transformé en le sel de potassium ou de sodium.

9. Utilisation de 1(S)-phényl-1,2,3,4-tétrahydroisoquinoline (II), en particulier de 1(S)-phényl-1,2,3,4-tétrahydroisoquinoline (II) optiquement enrichie ou optiquement pure, pouvant être obtenue conformément au procédé selon la revendication 7 pour la préparation de solifénacine ou d'un sel pharmaceutiquement acceptable de celle-ci.

10. Utilisation de 1-phényl-1,2,3,4-tétrahydroisoquinoline (III) potassique ou sodique, en particulier de 1(S)-phényl-1,2,3,4-tétrahydroisoquinoline (III) potassique ou sodique optiquement enrichie ou optiquement pure, pour la préparation de solifénacine ou d'un sel pharmaceutiquement acceptable de celle-ci.
